# EUROPÄISCHE PATENTSCHRIFT

(11) **EP 1 740 129 B1**
(45) Veröffentlichungstag und Bekanntmachung des Hinweises auf die Patenterteilung: **25.03.2020**
(21) Anmeldenummer: 04729392.3
(22) Anmeldetag: 26.04.2004
(51) Int. Cl.: A61F 2/44

(54) **ZWISCHENWIRBELPROTHESE ODER BANDSCHEIBENPROTHESE**
INTERVERTEBRAL PROSTHESIS OR DISK PROSTHESIS
PROTHESE INTERVERTEBRALE OU PROTHESE DE DISQUE

(43) Veröffentlichungstag der Anmeldung: 10.01.2007
(73) Patentinhaber: Synthes GmbH, 4436 Oberdorf (CH)
(72) Erfinder: LECHMANN, Beat, CH-2544 Bettlach (CH); FRIGG, Robert, CH-2544 Bettlach (CH); BÜRKI, Roger, CH-4710 Balsthal (CH)
(74) Vertreter: Orr, Robert
(86) Internationale Anmeldenummer: PCT/CH2004/000250
(87) Internationale Veröffentlichungsnummer: WO 2005/102226

(56) Entgegenhaltungen:
- WO-A-01/56513
- WO-A-97/37619
- WO-A-02/078514
- WO-A1-00/66044
- WO-A2-03/071992
- US-A1- 2001 032 018

## Beschreibung

Die Erfindung bezieht sich auf eine Zwischenwirbelprothese oder Bandscheibenprothese gemäss dem Oberbegriff des Patentanspruchs 1, insbesondere für die Arthrodesen-Chirurgie mittels dorsalem Zugang PLIF (posterior lumbar interbody fusion), TLIF (transforaminal lumbar interbody fusion), ELIF (extraforaminal lumbar interbody fusion), ALIF (anterior lumbar interbody fusion) und ACIF (anterior cervical interbody fusion). Diese Operationstechnik verfolgt das Ziel der Behandlung einer degenerierten oder anderweitig erkrankten Bandscheibe. Der Chirurg sucht den Zugang zur Bandscheibe durch einen mittig angelegten Hautschnitt. Anschliessend legt er den hinteren Bereich der Bewegungssegmente, insbesondere die Laminae und die Pedikeleintrittspunkte frei. Mittels teilweiser Resektion der fazettären und laminären Bestandteile der betroffenen Wirbelkörper zielt der Chirurg an den Nervenwurzeln und am Markraum vorbei hin zur erkrankten Bandscheibe.
Bei dieser Operationstechnik ist nur eine beschränkte Menge an autologer Spongiosa verfügbar, um damit die Hohlräume von käfigartigen Zwischenwirbel- oder Bandscheibenprothese und die Zwischenräume zwischen einzelnen Implantaten und ihrer Umgebung zu füllen. Langfristig erfolgt die Arthrodesis nicht mit dem Implantat sondern zwischen dem Knochen und dem Knochenersatzmaterial. Die einzelnen Implantate dienen somit nur als Platzhalter oder Abstandhalter.

Die mit den bekannten Zwischenwirbelimplantaten versorgten Zwischenwirbelräume gelangen oft nicht zur kompletten Arthrodese, d.h. sie enden in einer Pseudoarthrose. Ähnlich verhält es sich auch bei an käfigartigen Zwischenwirbelimplantaten für die Halswirbelsäule sowie solchen die durch ventrale Zugänge eingesetzt werden. Solche Zwischenwirbelräume sind mechanisch nicht stabil, wie man es von einer Versteifung erwarten würde. Rückkehrende Schmerzen mit nachfolgender Revisions-Chirurgie können dann die Folgen sein.

Bei den oben beschriebenen bekannten Implantaten und Operationstechniken verwendet der Chirurg autologes Knochenmaterial, das er von den resezierten Wirbelkörperteilen oder durch einen zusätzlichen Eingriff am Beckenkamm gewinnt. Da dorsale Zugänge zum Bandscheibenraum sehr eng sind, ist das Anlegen des Knochenmaterials erschwert. Der Chirurg kann nicht sicherstellen, dass der gesamte Zwischenwirbelraum mit autologem Knochenmaterial ausgefüllt ist. Es besteht somit die Gefahr, dass Leerräume entstehen, was einerseits das Wandern (Migrieren) des Implantates erlaubt und anderseits werden die nicht mit autologem Knochenmaterial gefüllten Räume durch eine weiches, fibröses Gewebe gefüllt.

Hier will die Erfindung Abhilfe schaffen. Der Erfindung liegt die Aufgabe zugrunde, eine Zwischenwirbelprothese oder Bandscheibenprothese zu schaffen, welche einen asymmetrischen Austritt des Knochenzementes ermöglicht, so dass einzelne Regionen zwischen den Wirbelkörpern (z.B. die gegen zentral und posterior gelegenen Zonen) automatisch mit mehr Knochenzement versorgt werden als andere Regionen.

WO 03/071992 beschreibt ein Implantat nach dem Oberbegriff von Anspruch 1. Weitere bekannte Implantate werden in WO 00/66044, WO 97/37619 und WO 02/078514 beschrieben.

Die Aufgabe wird durch ein Implantat aus zwei nebeneinander angeordneten Zwischenwirbelprothesen oder Bandscheibenprothesen in Form von Hohlkörpern mit jeweils einer vorderen Seite, einer hinteren Seite, einer zur Anlage an die Grundplatte eines Wirbelkörpers geeigneten oberen Seite, einer zur Anlage an die Grundplatte eines Wirbelkörpers geeigneten unteren Seite, einer rechten Seite, einer linken Seite, einem zur Aufnahme von fliessfähigem Knochenzement geeigneten Hohlraum, einer Eintrittsöffnung in den Hohlraum und mehreren Austrittsöffnungen für fliessfähigen Knochenzement aus dem Hohlraum gelöst, wobei für jede der zwei Zwischenwirbelprothesen oder Bandscheibenprothesen die Summe der Querschnittsflächen der aus der vorderen Seite austretenden Austrittsöffnungen S_{V}, die Summe der Querschnittsflächen der aus der hinteren Seite austretenden Austrittsöffnungen S_{H}, die Summe der Querschnittsflächen der aus der rechten Seite austretenden Austrittsöffnungen S_{R} und die Summe der Querschnittsflächen der aus der linken Seite austretenden Austrittsöffnungen S_{L} beträgt, und wobei
A) die rechte Seite der links angeordneten Zwischenwirbelprothese bzw. Bandscheibenprothese zur linken Seite der rechts angeordneten Zwischenwirbelprothese bzw. Bandscheibenprothese hin orientiert ist; und
B) für die links angeordnete Zwischenwirbelprothese bzw. Bandscheibenprothese die Bedingung S_{L} > S_{R} und für die rechts angeordnete Zwischenwirbelprothese bzw. Bandscheibenprothese S_{R} > S_{L} gilt;
   dadurch gekennzeichnet, dass
C) die rechte Seite jeder Zwischenwirbelprothese bzw. Bandscheibenprothese gerade ist und eine erste sich zwischen der vorderen Seite und der hinteren Seite der Zwischenwirbelprothese bzw. Bandscheibenprothese erstreckenden Länge hat, die linke Seite jeder Zwischenwirbelprothese bzw. Bandscheibenprothese gerade ist und eine zweite sich zwischen der vorderen Seite und der hinteren Seite der Zwischenwirbelprothese bzw. Bandscheibenprothese erstreckenden Länge hat, und die erste Länge gleich der zweiten Länge ist.

Anders ausgedrückt sind die Austrittsöffnungen derart dimensioniert, dass bei einer Zufuhr von fliessfähigem Knochenzement durch die Eintrittsöffnung in den Hohlraum entweder die durch S_{L} austretende Knochenzementmenge K_{L} grösser oder kleiner ist als die durch S_{R} austretende Knochenzementmenge K_{R} oder die durch S_{H} austretende Knochenzementmenge K_{H} ist grösser oder kleiner als die durch S_{V} austretende Knochenzementmenge K_{V}.

Die Erfindung erlaubt das Füllen des Zwischenwirbelraumes mit synthetischem Knochenmaterial (Knochenzement) nach erfolgter Plazierung der käfigartigen Zwischenwirbelprothese oder Bandscheibenprothese. Durch das Austreten und nachfolgende Aushärten des fliessfähigen, hydraulischen Knochenzementes wird das Implantat gesichert. Durch die asymmetrische Anordnung der Austrittsöffnungen im Implantat kann dar Knochenzement gezielt verbreitet werden. Die erfindungsgemässe Prothese hat weiter den Vorteil, dass sie eine zusätzliche Knochenentnahme am

Beckenkamm des Patienten, welche lang anhaltende Schmerzen verursacht, überflüssig macht.

Bei einer besonderen Ausführungsform ist die Eintrittsöffnung in der vorderen Seite der Prothese angebracht und der Hohlraum verläuft von der Eintrittsöffnung aus in Richtung der hinteren Seite.

Bei einer weiteren Ausführungsform ist die Eintrittsöffnung in der linken oder rechten Seite der Prothese angebracht und der Hohlraum verläuft von der Eintrittsöffnung aus in Richtung der gegenüber liegenden rechten oder linken Seite.

Bei einer weiteren Ausführungsform verkleinert sich der Querschnitt des Hohlraums mindesten auf einer Teilstrecke mit zunehmender Entfernung von der Eintrittsöffnung. Durch die Verjüngung des Hohlraums fliesst die flüssige Zementmischung einfacher durch die seitlichen Öffnungen des Implantates. Die in der Öffnung jeweils dem Einspritzpunkt gegenüberliegende Wand des Implantates wirkt wie eine Abscherkante, so dass die flüssige Zementmischung umgeleitet wird.

Bei einer weiteren Ausführungsform verjüngt sich der Hohlraum, mindestens auf einer Teilstrecke, entweder keilförmig oder konisch. Bei einer weiteren Ausführungsform konvergieren die obere und untere Seite gegen die vordere Seite hin mindestens auf einer Teilstrecke. Bei einer weiteren Ausführungsform ist die Prothese mindestens teilweise mit einem ausgehärteten hydraulischen Knochenzement gefüllt, der sich mindestens teilweise über die Austrittsöffnungen hinaus erstreckt.

Bei einer weiteren Ausführungsform kann das Implantat aus zwei nebeneinander angeordneten Zwischenwirbelprothesen oder Bandscheibenprothesen bestehen, wobei die rechte Seite der links angeordneten Zwischenwirbelprothese zur linken Seite der rechts angeordneten Zwischenwirbelprothese hin orientiert ist. Für die links angeordnete Zwischenwirbelprothese gilt die Bedingung S_{L}> S_{R} und für die rechts angeordnete Zwischenwirbelprothese die Bedingung S_{R}> S_{L}.

Die Zwischenwirbelprothese kann im übrigen in vielfältigster Weise variiert werden, z.B. durch Verwendung von planen, konkaven, konvexen oder auch kalottenförmigen Seitenwänden.

Als fliessfähige, hydraulische Knochenzemente eigenen sich insbesondere Kalziumphosphatzemente, die nach Vermischen der beiden Komponenten in flüssiger Form in das Implantat eingespritzt werden können und anschliessend hydraulisch aushärten.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht eines erfindungsgemässen linsenförmigen Zwischenwirbelimplantats;
Fig. 2 einen Längsschnitt durch das Zwischenwirbelimplantat nach Fig. 1 längs der Mittelebene VIII-VIII;
Fig. 3 eine Seitenansicht von rechts auf das Zwischenwirbelimplantat nach Fig. 1;
Fig. 4 eine Seitenansicht von links auf das Zwischenwirbelimplantat nach Fig. 1;
Fig. 5 eine perspektivische Ansicht einer erfindungsgemässen Zwischenwirbelprothese, welche mittels ausgehärtetem Knochenzement gesichert ist;
Fig. 6 eine Aufsicht auf die Zwischenwirbelprothese nach Fig. 5;
Fig. 7 eine perspektivische Ansicht einer Ausführungsvariante unter Verwendung von zwei Zwischenwirbelimplantaten, wobei der Knochenzement die Implantate sowohl gegeneinander in ihrer Position wie auch gegen ein Auseinanderwandern sichert;
Fig. 8 eine Aufsicht auf die beiden Zwischenwirbelimplantate nach Fig. 7;
Fig. 9 eine Vorderansicht einer Ausführungsvariante, bei der das perforierte Zwischenwirbelimplantat einen rechteckigen Querschnitt aufweist; und
Fig. 10 eine Vorderansicht einer Ausführungsvariante, bei der das perforierte Zwischenwirbelimplantat einen kreisförmigen Querschnitt aufweist.

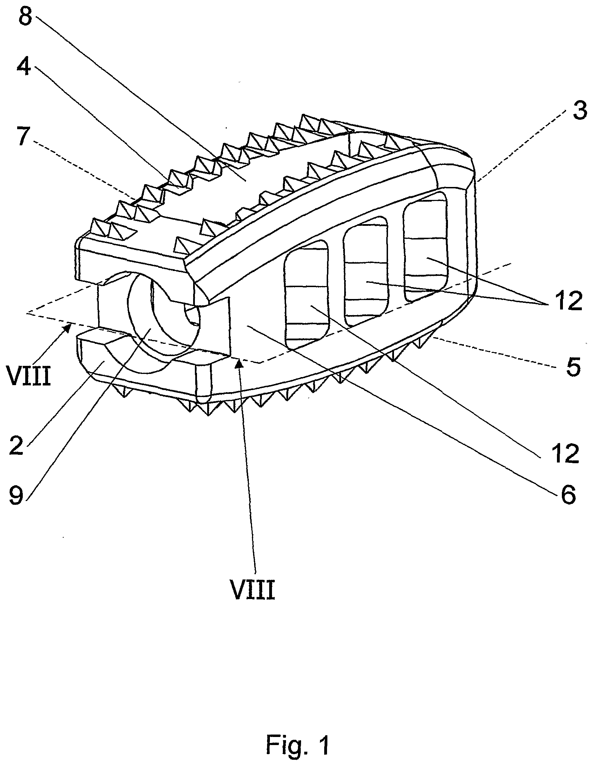

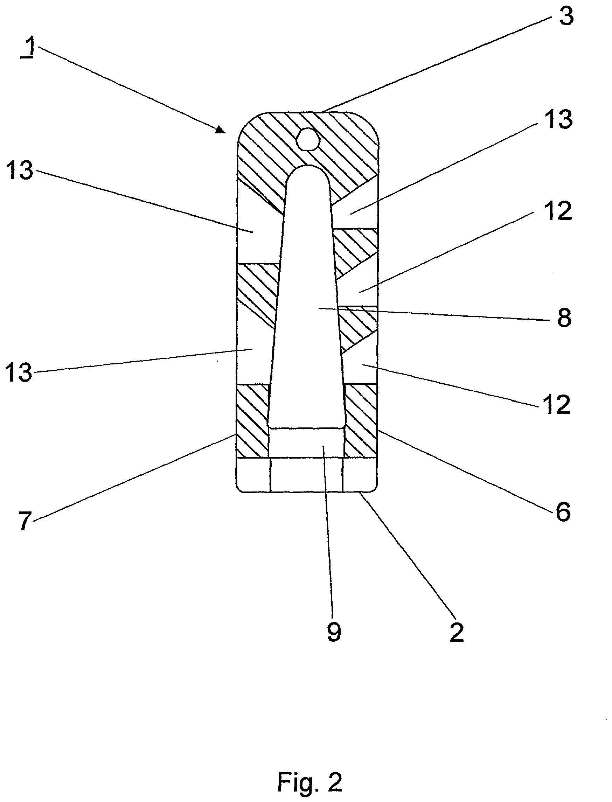

Die in den Fig. 1 und 2 dargestellt Zwischenwirbelprothese 1 besteht aus einem quaderförmigen Hohlkörper und besitzt eine vordere Seite 2, eine hintere Seite 3, eine zur Anlage an die Grundplatte eines Wirbelkörpers geeignete obere Seite 4, eine zur Anlage an die Grundplatte eines Wirbelkörpers geeignete untere Seite 5, eine rechte Seite 6, eine linke Seite 7, einen zur Aufnahme eines fliessfähigen, hydraulischen Knochenzements geeigneten Hohlraum 8, eine Eintrittsöffnung 9 in den Hohlraum 8 und mehreren Austrittsöffnungen 10;11;12;13 aus dem Hohlraum 8 auf. Die obere Seite 4 und die untere Seite 5 konvergieren sowohl gegen die vordere Seite 2 hin als auch gegen die hintere Seite 3 hin, so dass sich eine linsenähnliche Gestalt der Zwischenwirbelprothese 1 ergibt.

Wie aus Fig. 2 ersichtlich verkleinert sich der Querschnitt des Hohlraums 8 mit zunehmender Entfernung von der Eintrittsöffnung 9 in Form eines Konus.

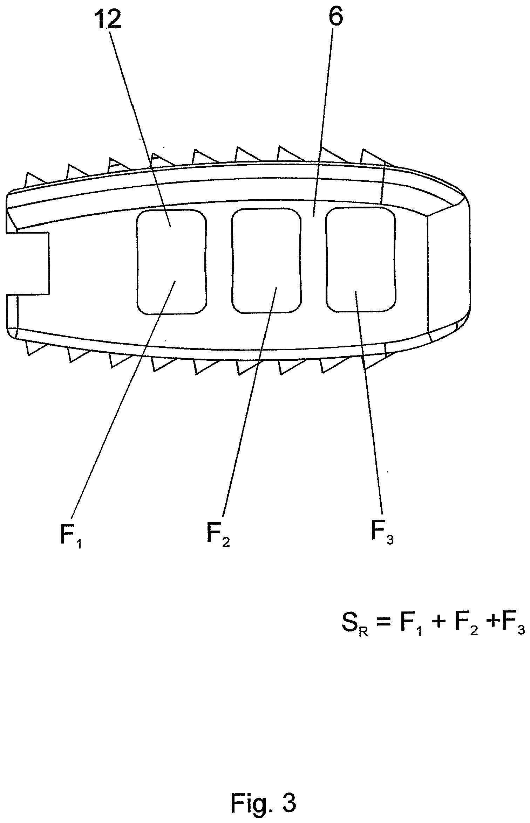

Wie in Fig. 3 dargestellt treten aus der rechten Seite 6 der Zwischenwirbelprothese 1 drei Austrittsöffnungen 12 mit den Flächen F₁ , F₂ und F₃ aus, so dass die Summe S_{R} der Querschnittsflächen der aus der rechten Seite 6 austretenden Austrittsöffnungen S_{R} = F₁ + F₂ + F₃ beträgt.

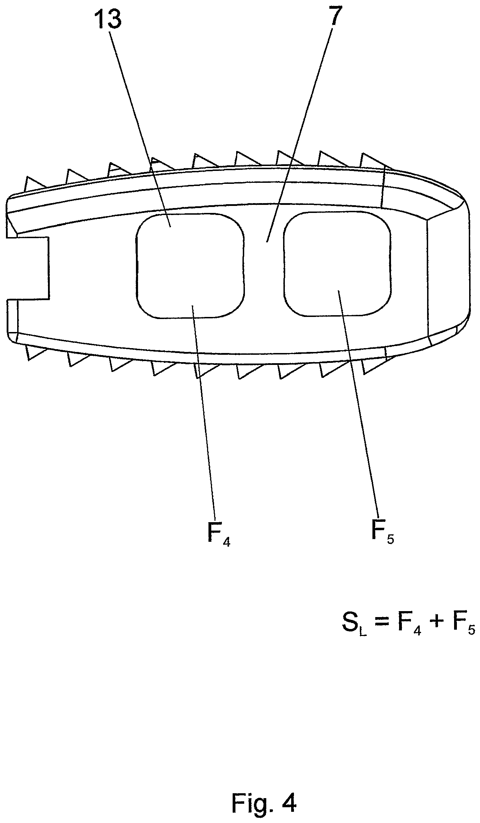

Wie in Fig. 4 dargestellt treten aus der linken Seite 7 der Zwischenwirbelprothese 1 zwei Austrittsöffnungen 13 mit den Flächen F₄ und F₅ aus, so dass die Summe S_{L} der Querschnittsflächen der aus der linken Seite 7 austretenden Austrittsöffnungen S_{L} = F₄ + F₅ beträgt.

Wesentlich ist, dass die Summe S_{L}> S_{R} ist, so dass auf der linken Seite 7 der Zwischenwirbelprothese 1 mehr Knochenzement aus dem Hohlraum 8 durch die Austrittsöffnungen 13 in den Zwischenwirbelraum austreten kann als auf der rechten Seite 6.

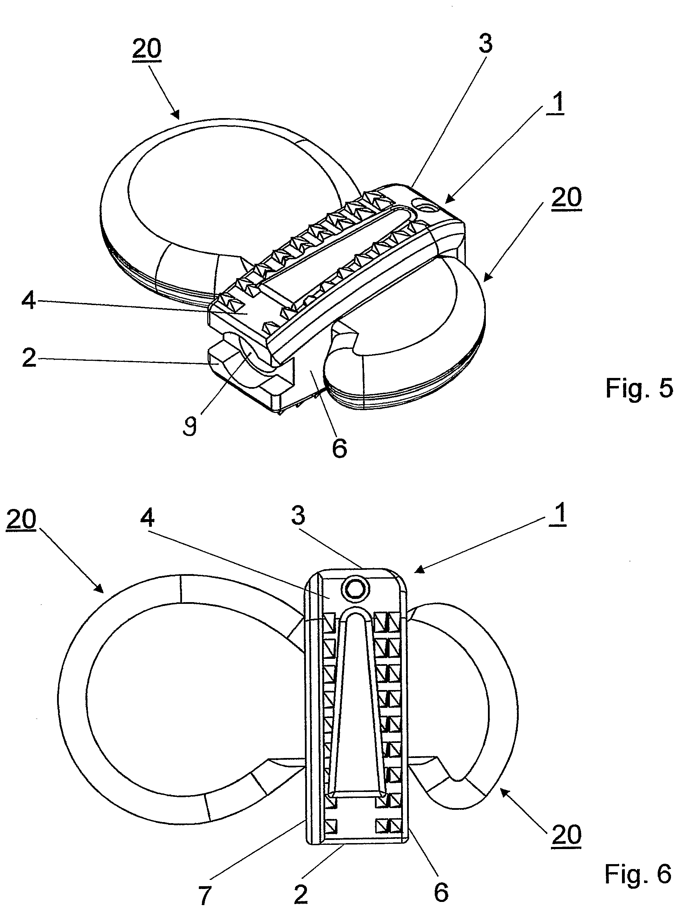

In den Fig. 5 und 6 ist dargestellt, wie sich der aus rechten Seite 6 und aus der linken Seite 7 der Zwischenwirbelprothese 1 austretende Knochenzement 20 verteilt, wobei wegen der grösseren Summe S_{L} der Querschnittsflächen der aus der linken Seite 7 austretenden Austrittsöffnungen 13 auch eine grösseren Menge Knochenzement 20 auf der linken Seite 7 austritt und aushärtet als auf der rechten Seite 6.

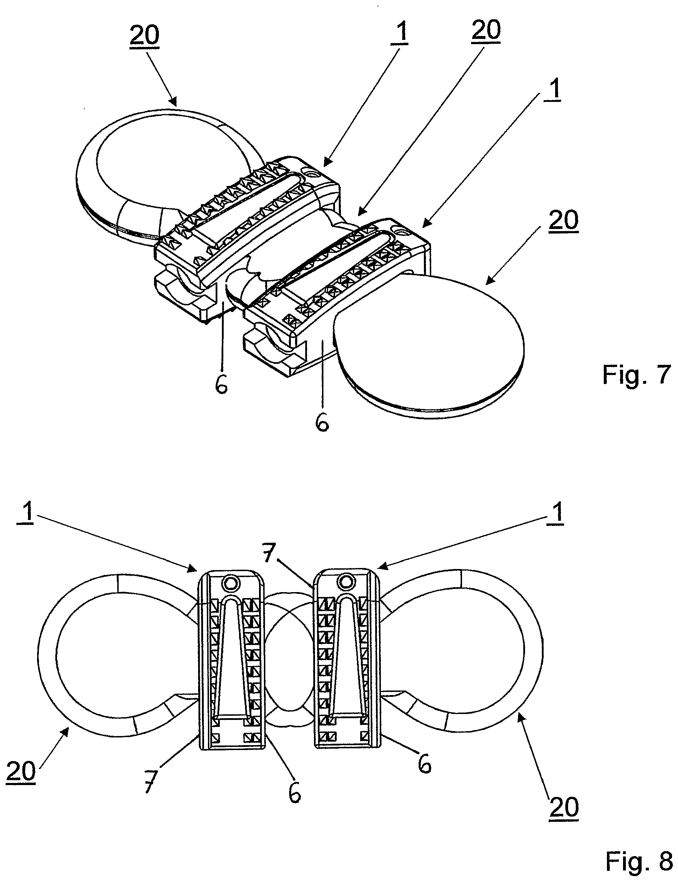

In den Fig. 7 und 8 ist eine weitere Ausführungsform dargestellt, welche aus zwei, nebeneinander angeordneten, erfindungsgemässen Zwischenwirbelprothesen 1 besteht. Dabei sind die beiden Zwischenwirbelprothesen 1 derart positioniert, dass die rechte Seite 6 der links angeordneten Zwischenwirbelprothese 1 zur linken Seite 7 der rechts angeordneten Zwischenwirbelprothese 1 hin orientiert ist. Für die links angeordnete Zwischenwirbelprothese 1 gilt die Bedingung S_{L} > S_{R}, währenddem für die rechts angeordnete Zwischenwirbelprothese 1 das umgekehrte, nämlich S_{R} > S_{L} gilt. Durch diese Massnahme tritt im Zwischenraum zwischen den beiden Zwischenwirbelprothese 1 weniger Knochenzement 20 aus im Vergleich zur rechten Seite 6 der rechts angeordneten Zwischenwirbelprothese 1 und im Vergleich zur linken Seite 7 der links angeordneten Zwischenwirbelprothese 1.

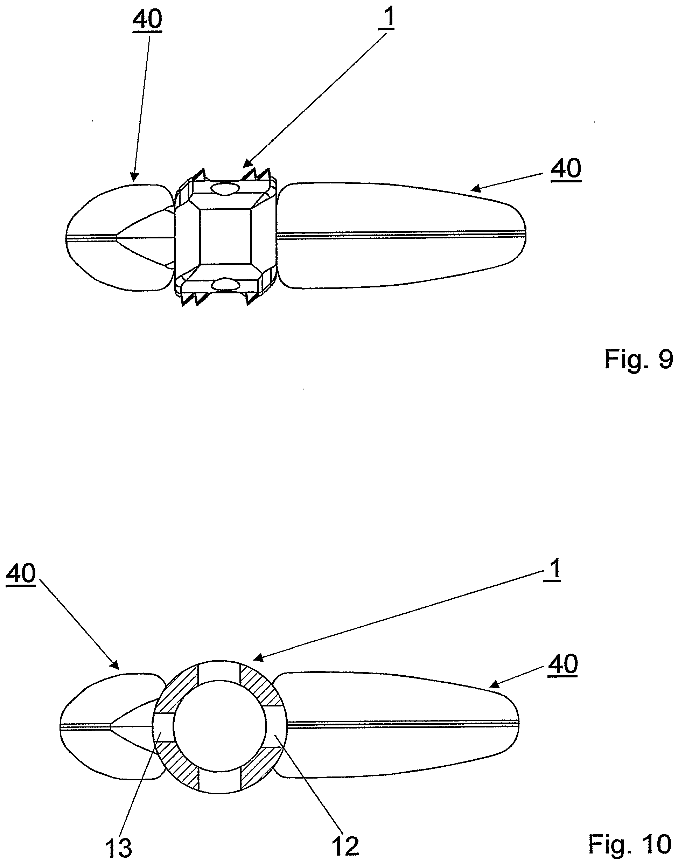

In Fig. 9 ist eine Ausführungsvariante einer erfindungsgemässen Zwischenwirbelprothese 1 dargestellt, welche einen rechteckigen Querschnitt aufweist und bei welcher auf der rechten Seite eine grössere Menge Knochenzement 40 ausgetreten ist als auf der linken Seite.

In Fig. 10 ist eine weitere Ausführungsvariante einer erfindungsgemässen Zwischenwirbelprothese 1 dargestellt, welche einen kreisförmigen Querschnitt aufweist und bei welcher auf der rechten Seite durch die Austrittsöffnungen 12 eine grössere Menge Knochenzement 40 ausgetreten ist als auf der linken Seite durch die Austrittsöffnungen 13.

## Patentansprüche

1. Implantat aus zwei nebeneinander angeordneten Zwischenwirbelprothesen oder Bandscheibenprothesen (1) in Form von Hohlkörpern mit jeweils einer vorderen Seite (2), einer hinteren Seite (3), einer zur Anlage an die Grundplatte eines Wirbelkörpers geeigneten oberen Seite (4), einer zur Anlage an die Grundplatte eines Wirbelkörpers geeigneten unteren Seite (5), einer rechten Seite (6), einer linken Seite (7), einem zur Aufnahme von fliessfähigem Knochenzement (20) geeigneten Hohlraum (8), einer Eintrittsöffnung (9) in den Hohlraum (8) und mehreren Austrittsöffnungen (10;11;12;13) für fliessfähigen Knochenzement aus dem Hohlraum (8), wobei für jede der zwei Zwischenwirbelprothesen oder Bandscheibenprothesen die Summe der Querschnittsflächen der aus der vorderen Seite (2) austretenden Austrittsöffnungen (10) Sv, die Summe der Querschnittsflächen der aus der hinteren Seite (3) austretenden Austrittsöffnungen (11) S_{H}, die Summe der Querschnittsflächen der aus der rechten Seite (6) austretenden Austrittsöffnungen (12) S_{R} und die Summe der Querschnittsflächen der aus der linken Seite (7) austretenden Austrittsöffnungen (13) S_{L} beträgt, und wobei
A) die rechte Seite (6) der links angeordneten Zwischenwirbelprothese bzw. Bandscheibenprothese (1) zur linken Seite (7) der rechts angeordneten Zwischenwirbelprothese bzw. Bandscheibenprothese (1) hin orientiert ist; und
B) für die links angeordnete Zwischenwirbelprothese bzw. Bandscheibenprothese (1) die Bedingung S_{L} > S_{R} und für die rechts angeordnete Zwischenwirbelprothese bzw. Bandscheibenprothese (1) S_{R} > S_{L} gilt;
**dadurch gekennzeichnet, dass**
C) die rechte Seite (6) jeder Zwischenwirbelprothese bzw. Bandscheibenprothese (1) gerade ist und eine sich zwischen der vorderen Seite und der hinteren Seite der Zwischenwirbelprothese bzw. Bandscheibenprothese erstreckenden erste Länge hat, die linke Seite (7) jeder Zwischenwirbelprothese bzw. Bandscheibenprothese (1) gerade ist und eine sich zwischen der vorderen Seite und der hinteren Seite der Zwischenwirbelprothese bzw. Bandscheibenprothese erstreckenden zweite Länge hat, und die erste Länge gleich der zweiten Länge ist.

2. Implantat nach Anspruch 1, **dadurch gekennzeichnet, dass** die Zwischenwirbelprothesen oder Bandscheibenprothesen (1) einstückig sind.

3. Implantat nach Anspruch 1 oder 2, **dadurch gekennzeichnet, dass** für jede der zwei Zwischenwirbelprothesen oder Bandscheibenprothesen die Eintrittsöffnung (9) in der vorderen Seite (2) angebracht ist und der Hohlraum (8) von der Eintrittsöffnung (9) aus in Richtung der hinteren Seite (3) verläuft.

4. Implantat nach Anspruch 1 oder 2, **dadurch gekennzeichnet, dass** für jede der zwei Zwischenwirbelprothesen oder Bandscheibenprothesen die Eintrittsöffnung (9) in der linken oder rechten Seite (6,7) angebracht ist und der Hohlraum (8) von der Eintrittsöffnung (9) aus in Richtung der gegenüber liegenden rechten oder linken Seite (7,6) verläuft.

5. Implantat nach Anspruch 3 oder 4, **dadurch gekennzeichnet, dass** für jede der zwei Zwischenwirbelprothesen oder Bandscheibenprothesen sich der
Querschnitt des Hohlraums (8) mit zunehmender Entfernung von der Eintrittsöffnung (9), mindesten auf einer Teilstrecke, verkleinert.

6. Implantat nach Anspruch 5, **dadurch gekennzeichnet, dass** für jede der zwei Zwischenwirbelprothesen oder Bandscheibenprothesen sich der Hohlraum (8) - mindestens auf einer Teilstrecke- keilförmig oder konisch verjüngt.

7. Implantat nach einem der Ansprüche 1 bis 6, **dadurch gekennzeichnet, dass** für jede der zwei Zwischenwirbelprothesen oder Bandscheibenprothesen die obere (4) und untere Seite (5) gegen die vordere Seite (2) hin mindestens auf einer Teilstrecke konvergieren.

8. Implantat nach einem der Ansprüche 1 bis 7, **dadurch gekennzeichnet, dass** jede der zwei Zwischenwirbelprothesen oder Bandscheibenprothesen
mindestens teilweise mit einem ausgehärteten, hydraulischen Knochenzement (20) gefüllt ist, der sich mindestens teilweise über die Austrittsöffnungen (10; 11; 12; 13) der jeweiligen Zwischenwirbelprothese oder Bandscheibenprothese hinaus erstreckt.

9. Implantat nach einem der Ansprüche 1 bis 8, **dadurch gekennzeichnet, dass** zwischen den zwei nebeneinander angeordneten Zwischenwirbelprothesen oder Bandscheibenprothesen (1) ein Zwischenraum angeordnet ist.

## Claims

1. An implant comprising two intervertebral or disk prostheses, arranged beside each other, in the form of hollow bodies, each having front side (2), a rear side (3), a top side (4) for contacting the base plate of a vertebral body, a bottom side (5) for contacting the base plate of a vertebral body, a right side (6), a left side (7), a cavity (8) for receiving fluid osteocementum (20), an inlet (9) into the cavity (8) and several outlets (10, 11, 12, 13) for flowable osteocementum out of the cavity (8), wherein for each of the two intervertebral or disk prostheses the sum of the cross sectional areas of the outlets (10) from the front side (2) is S_{V}, the sum of the cross sectional areas of the outlets (11) from the rear side (3) is S_{H}, the sum of the cross sectional areas of the outlets (12) from the right side (6) is S_{R}, and the sum of the cross sectional areas of the outlets (13) from the left side (7) is S_{L}, and wherein
A) the right side (6) of the intervertebral or disk prosthetic (1) arranged on the left is oriented towards the left side (7) of the intervertebral or disk prosthetic (1) arranged on the right; and
B) for the intervertebral or disk prosthetic (1) arranged on the left the condition S_{L} > S_{R} applies and for the intervertebral or disk prosthetic (1) arranged on the right the condition S_{R} > S_{L} applies;
**characterised in that**
C) the right side (6) of each intervertebral or disk prosthetic (1) is straight and has a first length extending between the front side and the rear side of the intervertebral or disk prosthetic, the left side (7) of each intervertebral or disk prosthetic (1) is straight and has a second length extending between the front side and the rear side of the intervertebral or disk prosthetic, and the first length is equal to the second length.

2. An implant according to claim 1, **characterized in that** the intervertebral or disk prostheses are in one piece.

3. An implant according to claim 1 or 2, **characterized in that** for each of the two intervertebral or disk prostheses the inlet (9) is provided in the front side (2) and the cavity (8) extends from the inlet (8) towards the rear side (3).

4. An implant according to claim 1 or 2, **characterized in that** for each of the two intervertebral or disk prostheses the inlet (9) is provided in the left or right side (6, 7) and the cavity (8) extends from the inlet (9) towards the opposite right or left side (7, 6).

5. An implant according to claim 3 or 4, **characterised in that** for each of the two intervertebral or disk prostheses the cross section of the cavity (8) decreases with increasing distance from the inlet (9), at least over a partial distance.

6. An inlet according to claim 5, **characterised in that** for each of the two intervertebral or disk prostheses the cavity (8) - at least over a partial distance - tapers in a wedge-shaped or conical manner.

7. An implant according to one of claims 1 to 6, **characterised in that** for each of the two intervertebral or disk prostheses the top (4) and bottom (5) side converge towards the front side (2) at least over a partial distance.

8. An implant according to one of claims 1 to 7, **characterised in that** each of the two intervertebral or disk prostheses is at least partially filled with a hardened, hydraulic osteocementum (20), which extends at least partially beyond the outlets (10; 11; 12; 13) of the respective intervertebral or disk prosthetic.

9. An implant according to one of claims 1 to 8, **characterised in that** an intermediate space is arranged between the two intervertebral or disk prostheses arranged beside each other.

## Revendications

1. Implant composé de deux prothèses intervertébrales ou prothèses de disque (1) disposées côte à côte sous la forme de corps creux avec respectivement un côté avant (2), un côté arrière (3), un côté supérieur (4) adapté pour venir en appui au niveau de la plaque de base d'un corps vertébral, un côté inférieur (5) adapté pour venir en appui au niveau de la plaque de base d'un corps vertébral, un côté droit (6), un côté gauche (7), une cavité (8) adaptée pour recevoir du ciment osseux (20) fluide, un orifice d'entrée (9) dans la cavité (8) et plusieurs orifices de sortie (10 ; 11 ; 12 ; 13) pour du ciment osseux fluide menant hors de la cavité (8), dans lequel pour chacune des deux prothèses intervertébrales ou prothèses de disque, la somme des surfaces de section transversale des orifices de sortie (10) sortant du côté avant (2) est S_{V}, la somme des surfaces de section transversale des orifices de sortie (11) sortant du côté arrière (3) est S_{H}, la somme des surfaces de section transversale des orifices de sortie (12) sortant du côté droit (6) est S_{R} et la somme des surfaces de section transversale des orifices de sortie (13) sortant du côté gauche (7) est S_{L}, et dans lequel A) le côté droit (6) de la prothèse intervertébrale ou de la prothèse de disque (1) disposée à gauche est orienté en direction du côté gauche (7) de la prothèse intervertébrale ou de la prothèse de disque (1) disposée à droite ; et
B) la condition S_{L} > S_{R} s'applique pour la prothèse intervertébrale ou la prothèse de disque (1) disposée à gauche et la condition S_{R} > S_{L} s'applique pour la prothèse intervertébrale ou la prothèse de disque (1) disposée à droite ;
**caractérisé en ce que**
C) le côté droit (6) de chaque prothèse intervertébrale ou de prothèse de disque (1) est droit et a une première longueur s'étendant entre le côté avant et le côté arrière de la prothèse intervertébrale ou de la prothèse de disque, le côté gauche (7) de chaque prothèse intervertébrale ou prothèse de disque (1) est droit et a une deuxième longueur s'étendant entre le côté avant et le côté arrière de la prothèse intervertébrale ou de la prothèse de disque, et la première longueur est égale à la deuxième longueur.

2. Implant selon la revendication 1, **caractérisé en ce que** les prothèses intervertébrales ou prothèses de disque (1) sont d'un seul tenant.

3. Implant selon la revendication 1 ou 2, **caractérisé en ce que** pour chacune des deux prothèses intervertébrales ou prothèses de disque, l'orifice d'entrée (9) est pratiqué dans le côté avant (2), et la cavité (8) s'étend depuis l'orifice d'entrée (9) en direction du côté arrière (3).

4. Implant selon la revendication 1 ou 2, **caractérisé en ce que** pour chacune des deux prothèses intervertébrales ou prothèses de disque, l'orifice d'entrée (9) est pratiqué dans le côté gauche ou droit (6, 7) et la cavité (8) s'étend depuis l'orifice d'entrée (9) en direction du côté droit ou gauche (7, 6) se faisant face.

5. Implant selon la revendication 3 ou 4, **caractérisé en ce que** pour chacune des deux prothèses intervertébrales ou prothèses de disque, la section transversale de la cavité (8) se réduit au fur et à mesure que l'éloignement de l'orifice d'entrée (9) augmente, au moins sur un tronçon partiel.

6. Implant selon la revendication 5, **caractérisé en ce que** pour chacune des deux prothèses intervertébrales ou prothèses de disque, la cavité (8) se rétrécit de manière à présenter une forme de coin ou de manière conique au moins sur un tronçon partiel.

7. Implant selon l'une quelconque des revendications 1 à 6, **caractérisé en ce que** pour chacune des deux prothèses intervertébrales ou prothèses de disque, le côté supérieur (4) et le côté inférieur (5) convergent à l'encontre du côté avant (2) au moins vers un tronçon partiel.

8. Implant selon l'une quelconque des revendications 1 à 7, **caractérisé en ce que** chacune des deux prothèses intervertébrales ou prothèses de disque est remplie au moins en partie d'un ciment osseux (20) hydraulique durci, qui s'étend au moins en partie au-delà des orifices de sortie (10 ; 11 ; 12 ; 13) de la prothèse intervertébrale ou prothèse de disque respective.

9. Implant selon l'une quelconque des revendications 1 à 8, **caractérisé en ce qu'**un espace intermédiaire est disposé entre les deux prothèses intervertébrales ou prothèses de disque (1) disposées côte à côte.
